(19) Europäisches Patentamt
European Patent Office
Office européen des brevets

(11) Numéro de publication: **0 098 186**
**B1**

# (12) FASCICULE DE BREVET EUROPEEN

(45) Date de publication du fascicule du brevet: **16.09.87**

(51) Int. Cl.⁴: **A 61 K 31/70**, C 07 H 19/06, C 07 H 19/16

(21) Numéro de dépôt: **83401171.0**

(22) Date de dépôt: **09.06.83**

(54) **Compositions pharmaceutiques à base de xylosides et lyxosides de bases puriqes et pyrimidiques.**

(30) Priorité: **14.06.82 FR 8210289**

(43) Date de publication de la demande: **11.01.84 Bulletin 84/2**

(45) Mention de la délivrance du brevet: **16.09.87 Bulletin 87/38**

(84) Etats contractants désignés: **BE CH DE FR GB IT LI LU NL SE**

(56) Documents cités:
**JOURNAL OF MEDICINAL CHEMISTRY, vol. 19, no. 8, 1976, pages 1026-1028, USA**
**CIRCULATION RESEARCH, vol. 45, no. 4, octobre 1979, pages 468-478**
**CANCER CHEMOTHERAPY REPORTS, partie 2, vol. 3, no. 1, novembre 1972, pages 95-109**
**COLLECTION CZECHOSLOV. CHEM. COMMUN., vol. 43, 1978, pages 3279-3291**
**HELVETICA CHIMICA ACTA, vol. 61, fascicule 3, no. 90, 1978, pages 1011-1016**
**COLLECTION CZECHOSLOV. CHEM. COMMUN., vol. 43, 1978, pages 2330-2340**
**CHEMICAL ABSTRACTS, vol. 75, no. 11, 13 septembre 1971, page 502, no. 77232h, Columbus, Ohio, USA**

(73) Titulaire: **SYNTHELABO, 58, rue de la Glacière, F-75621 Paris Cedex 13 (FR)**
Titulaire: ***Imbach, Jean-Louis, 1108, rue de Las Sorbes,*** **F-34000 Montpellier (FR)**

(72) Inventeur: **Imbach, Jean-Louis, 1108, rue de Las Sorbes, F-34000 Montpellier (FR)**
Inventeur: **Gosselin, Gilles, Rés. "Parc des Arceaux" Bât. Fl, rue Paul Rimbaud, F-34000 Montpellier (FR)**
Inventeur: **De Rudder, Jean, 40, Avenue des Etats-Unis, F-78000 Versailles (US)**

(74) Mandataire: **Thouret-Lemaitre, Elisabeth et al, Service Brevets - SYNTHELABO 58, rue de la Glacière, F-75621 Paris Cédex 13 (FR)**

(56) References cited: (continuation)
**JOURNAL OF THE AMERICAN CHEMICAL SOCIETY, vol. 80, no. 19, 7 octobre 1958, pages 5155-5160**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne des compositions pharmaceutiques à base de xylosides et lyxosides de bases puriques et pyrimidiques.

Les médicaments de l'invention sont actifs notamment à l'égard des virus à ADN (acide désoxyribonucléique) tels que herpès, vaccine et adénovirus.

Les composés utilisés selon l'invention sont la β–D-xylofurannosyl-1 cytosine (ou β-xylo-C) et l'α–D-lyxofurannosyl-9 adénine (ou α-xylo-A).

Ces deux composés sont des substances connues et déjà décrites dans la littérature.

La β–D-xylofurannosyl-1 cytosine de formule

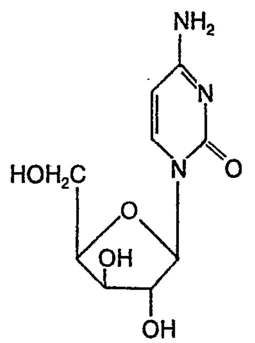

est une substance déjà connue et décrite par J. J. Fox et coll. (JACS. 79, p. 5060, 1957), et V. Brodbeck et coll. (J. Org. Chem. 35, 3552, 1970).

L'α–D-lyxofurannosyl-9 adénine a été décrite par P. Kohn et coll. (J. Org. Chem., 32, 4076 (1967).

Les β–D-xyloside et α–D-lyxoside ont été préparés selon des méthodes de condensations, choisies ponctuellement en fonction de la réactivité de l'aglycone.

La préparation de la β-xylo-C utilisée selon l'invention est décrite dans l'exemple suivant;

Les analyses et les spectres UV, de masse et de RMN ont confirmé la structure de ce composé.

EXEMPLE β–D-xylofurannosyl-1 cytosine

On condense un équivalent de la cytosine avec un équivalent d'O-acétyl-1 tri-o-benzoyl-2,3,5 α–D-xylofurannose, à la température ambiante, dans de l'acétonitrile anhydre, en présence de tétrachlorure d'étain $SnCl_4$ (2 eq); on purifie le composé obtenu par chromatographie sur colonne de silice et on débenzoyle à l'aide d'une solution de méthanol saturée d'ammoniac.

On obtient la β–D-xylofurannosyl-1 cytosine. F = 240–242 °C (dec)

Les deux composés utilisés selon l'invention possèdent des propriétés virostatiques et ont été étudiés in vitro dans les conditions suivantes:

on opère sur des cellules HeLa cultivées dans un milieu constitué par du liquide de Earle additionné de 0,5% d'hydrolysat de lactalbumine, 0,1% d'extrait de levure, 5% de sérum de veau filtré et inactivé trente minutes à 56 °C. On réalise la culture dans des tubes stationnaires contenant 1 ml de ce milieu. Les nappes cellulaires étant bien développées, on élimine le milieu de culture et introduit dans chaque tube 0,1 ml d'une suspension de virus herpétique dans du liguide de Hanks à une concentration suffisante pour contenir de 10 à 50 D.I.C.T.$_{50}$ (dose infectieuse en culture de tissu 50%) par cellule. On laisse absorber à 37 °C pendant deux heures. On élimine le surnageant, lave la couche cellulaire à l'aide d'un soluté isotonique tamponné. Puis on introduit 1 ml du milieu de maintien (milieu analogue) mais renfermant 2,5% de sérum auquel on ajoute préalablement la substance à tester à la concentration de $3{,}3 \times 10^{-4}$M. On fait également des cultures témoins ne contenant pas de composé actif et inoculées par le virus de l'herpès dans les mêmes conditions. Tous les tubes sont incubés à 35 °C à l'étuve. Lorsque les témoins montrent un effet cytopathogène complet, on titre le virus infectieux dans tous les tubes par la méthode de l'établissement de la dilution limite 50%. On constate une différence de 3 à 5 unités logarithmiques de base 10 entre les titres infectieux des tubes traités et des tubes témoins, ce qui révèle un blocage complet de la production de virus dans les tubes traités.

La β–D-xylofurannosyl-1 cytosine et l'α–D-lyxofurannosyl-9 adénine ont été plus particulièrement étudiées dans la recherche de l'activité sur le virus de l'herpès (H. hominis type 1) dans des cultures de cellules HeLa. Dans le lot témoin le titre du virus est d'environ $10^{6,5}$ ($DICT_{50}$/ml).

Lorsque l'on introduit la substance à tester le titre du virus diminue et est respectivement de $10^{3,6}$ et $10^{2,5}$ ($DICT_{50}$/ml).

La toxicité des composés à l'égard des cellules normales en culture est très faible.

En raison de leurs propriétés virostatiques et de leur faible toxicité à l'égard des cellules, les deux composés β-xylo-C et α-lyxo-A sont des substances utiles en thérapeutique pour le traitement de diverses viroses telles la kératine herpétique, l'herpès cutané, le zona ainsi que les maladies causées par les poxvirus et les adénovirus.

Les compositions pharmaceutiques de l'invention peuvent être liquides ou solides et se présenter sous la forme de solutions, collyres, onguents, pommades ...

Elles sont préparées selon les méthodes usuelles. Le principe actif peut y être incorporé à des excipients pharmaceutiques habituellement utilisés dans de telles compositions et choisis en fonction de la forme galénique à réaliser. Ces excipients peuvent être constitués par des solvants aqueux ou non, des stabilisants, des mouillants, des vaselines, des lanolines, des polyéthylène-glycols, des esters d'acides gras du sorbitanne, polyhydroxyéthylés ou non.

La concentration en principe actif des préparations pharmaceutiques ainsi réalisées est variable selon la gravité des cas et le mode d'utilisation envisagé: elle peut être par exemple comprise entre 0,1% et 5%.

## Revendications

1. Composition pharmaceutique caractérisée par le fait qu'elle contient de la β–D-xylofurannosyl-1 cytosine ou de l'α–D-lyxo-furannosyl-9 adénine.

2. Composition pharmaceutique ayant une activité à l'égard des virus à acide désoxyribonucléique, caractérisée par le fait qu'elle contient un des composés spécifiés dans la revendication 1.

3. Composition pharmaceutique à activité antiherpétique caractérisée par le fait qu'elle contient un des composés spécifiés dans la revendication 1.

4. Composition pharmaceutique à activité antiherpétique caractérisée par le fait qu'elle contient la β–D-xylofurannosyl-1 cytosine.

5. Composition pharmaceutique à activité antiherpétique caractérisée par le fait qu'elle contient l'α–D-lyxofurannosyl-9 adénine.

**Claims**

1. A pharmaceutical composition characterised in that it contains 1-(β–D-xylofuranosyl)-cytosine or 9-(α–D-lyxofuranosyl)-adenine.

2. Pharmaceutical composition which is active against deoxyribunocleic acid viruses characterised in that it contains one of the compounds specified in claim 1.

3. A pharmaceutical composition with antiherpes activity characterised in that it contains one of the compounds specified in claim 1.

4. A pharmaceutical composition with antiherpes activity characterised in that it contains 1-(β–D-xylofuranosyl)-cytosine.

5. A pharmaceutical composition with antiherpes activity characterised in that it contains 9-(α–D-lyxofuranosyl)-adenine.

**Patentansprüche**

1. Pharmazeutische Zubereitung, dadurch gekennzeichnet, dass sie 1-(β–D-xylofurannosyl)-cytosine oder 9-(α–D-lyxofurannosyl)-adenine enthält.

2. Pharmazeutische Zubereitung, die gegen Deoxyribonucleinsäure-Virus aktiv ist, dadurch gekennzeichnet, dass sie eine der Verbindungen des Anspruchs 1 enthält.

3. Pharmazeutische Zubereitung mit Antiherpes Aktivität, dadurch gekennzeichnet, dass sie eine der Verbindungen des Anspruchs 1 enthält.

4. Pharmazeutische Zubereitung mit Anitherpes Aktivität, dadurch gekennzeichnet, dass sie 1-(β–D-xylofurannosyl)-cytosine enthält.

5. Pharmazeutische Zubereitung mit Antiherpes Aktivität, dadurch gekennzeichnet, dass sie 9-(α–D-lyxofurannosyl)-adenine enthält.